# EUROPEAN PATENT APPLICATION

(11) **EP 4 470 469 A1**
(43) Date of publication of application: **04.12.2024**
(21) Application number: 23176368.1
(22) Date of filing: 31.05.2023
(51) Int. Cl.: A61B 6/00, A61B 6/03, A61B 6/04

(54) **POSITIONING OF AN IMAGING DEVICE**

(71) Applicant: Koninklijke Philips N.V., 5656 AG Eindhoven (NL)
(72) Inventor: HOMAN, Robert Johannes Frederik, Eindhoven (NL)
(74) Representative: Philips Intellectual Property & Standards

(57) **Abstract**

The present invention relates to positioning an imaging device in a dual-modality imaging system. In order to provide improved positioning of an imaging device during a medical procedure, a device (10) for positioning an imaging device in a dual-modality imaging system is provided. The device comprises a data input (12), a data processor (14) and an output interface (16). The data input is configured: to receive first image data (18) of a subject from a first X-ray imaging modality; to receive at least one procedural parameter (20) relating to a current interventional medical procedure of the subject; and to provide a spatial registration (22) of the first X-ray imaging modality and a second X-ray imaging modality, the second X-ray imaging modality being different than the first X-ray imaging modality. The data processor is configured: to determine anatomical parameters of the subject in the first image data; and to calculate positional data (24) for the second X-ray imaging modality for generating current image data as second image data of the subject with the second imaging modality, wherein the positional data is based on the determined anatomical parameters, the procedural parameter and the provided spatial relation. The output interface is configured: to provide the calculated positional data for generating the second image data of the subject by the second X-ray imaging modality.

## Description

### FIELD OF THE INVENTION

The present invention relates to a device for positioning an imaging device in a dual-modality imaging system, to a medical dual-modality imaging system for generating first and second type of X-ray image data of a subject and to a method for determining imaging parameters.

### BACKGROUND OF THE INVENTION

During medical procedures, e.g. examinations or interventions, different imaging modalities may be used depending on different requirements or needs and the technical capabilities of the respectively available imaging systems. As an example, X-ray CT-imaging provides detailed 3D information. As another example, X-ray fluoroscopy imaging provides 2D images. However, it has been shown that in order to provide useful image data, e.g. during a procedure, proper positioning of an imaging system may be cumbersome and may hamper with the workflow of the medical procedure.

### SUMMARY OF THE INVENTION

There may thus be a need to provide improved positioning of an imaging device during a medical procedure.

The object of the present invention is solved by the subject-matter of the independent claims; further embodiments are incorporated in the dependent claims. It should be noted that the following described aspects of the invention apply also for the device for positioning an imaging device in a dual-modality imaging system, for the medical dual-modality imaging system for generating first and second type of X-ray image data of a subject and for the method for determining imaging parameters.

According to the present invention, a device for positioning an imaging device in a dual-modality imaging system is provided. The device comprises a data input, a data processor and an output interface. The data input is configured to receive first image data of a subject from a first X-ray imaging modality. The data input is also configured to receive at least one procedural parameter relating to a current interventional medical procedure of the subject. The data input is further configured to provide a spatial registration of the first X-ray imaging modality and a second X-ray imaging modality, the second X-ray imaging modality being different than the first X-ray imaging modality. The data processor is configured to determine anatomical parameters of the subject in the first image data. The data processor is configured to calculate positional data for the second X-ray imaging modality for generating current image data as second image data of the subject with the second imaging modality. The positional data is based on the determined anatomical parameters, the procedural parameter and the provided spatial relation. The output interface is configured to provide the calculated positional data for generating the second image data of the subject by the second X-ray imaging modality.

As an effect, a positioning for the second imaging modality is facilitated, thus improving the workflow for the user and also the subject.

According to an example, the first image data is X-ray CT imaging data. The second image data is X-ray C-arm imaging data. The positional data comprises position data of an X-ray C-arm imaging device serving as the second imaging modality.

As an advantage, X-ray imaging with a C-arm, such as fluoroscopy imaging can be provided in an efficient and dose saving way due to the facilitated positioning.

According to an example, the at least one procedural parameter comprises information about a selected or current medical procedure.

According to an example, the calculated positional data for the second imaging modality comprises at least one imaging position for an imaging procedure according to the defined procedural parameter.

According to an example, a database is provided with stored predefined optimized viewing directions for selected medical procedures and selected anatomical scenarios.

According to an example, the anatomical parameters are determined based on a segmentation of the first image data.

In an option, the data processor is configured to segment the first image data.

According to the present invention, also a medical dual-modality imaging system for generating first and second type of X-ray image data of a subject is provided. The system comprises a first X-ray imaging modality, a second X-ay imaging modality and a device according to one of the preceding examples. The first X-ray imaging modality and the second X-ray imaging modality provide different image acquisition techniques. The first X-ray imaging modality provides the first image data of the subject. The second X-ray imaging modality is configured to generate the second image data based on the calculated positional data.

According to an example, the first imaging modality is an X-ray CT imaging device with a movable gantry. The second imaging modality is a C-arm X-ray imaging device with a movably mounted C-arm.

According to an example, a common spatial coordinate system is provided to which the X-ray CT imaging device and the C-arm X-ray imaging device are linked.

According to the present invention, also a method for determining imaging parameters is provided. The method comprises the following steps:
- receiving first image data of a subject from a first X-ray imaging modality;
- determining anatomical parameters of the subject in the first image data;
- receiving at least one procedural parameter relating to a current interventional medical procedure of the subject;
- providing a spatial registration of the first X-ray imaging modality and a second X-ray imaging modality, the second X-ray imaging modality being different than the first X-ray imaging modality;
- calculating positional data for the second X-ray imaging modality for generating current image data with the second imaging modality; wherein the positional data is based on the determined anatomical parameters, the procedural parameter and the provided spatial relation; and
- providing the calculated positional data for generating second image data of the subject by the second X-ray imaging modality.

According to an aspect, image data from one imaging system is used to assess a current situation in order to then determine positioning data for the other imaging system based on a known spatial relationship of both imaging systems. In an example, image data from an X-ray CT imaging system is used to calculate positioning data for an X-ray C-arm imaging system.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for positioning an imaging device in a dual-modality imaging system.
Fig. 2 shows an example of a medical dual-modality imaging system for generating first and second type of X-ray image data of a subject.
Fig. 3 shows another example of the medical dual-modality imaging system with the first imaging modality being an X-ray CT imaging device with a movable gantry and the second imaging modality being a C-arm X-ray imaging device with a movably mounted C-arm.
Fig. 4 shows a perspective view of another example of the medical dual-modality imaging system with an X-ray CT imaging device and a C-arm X-ray imaging device.
Fig. 5 shows basic steps of an example of a method for determining imaging parameters.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

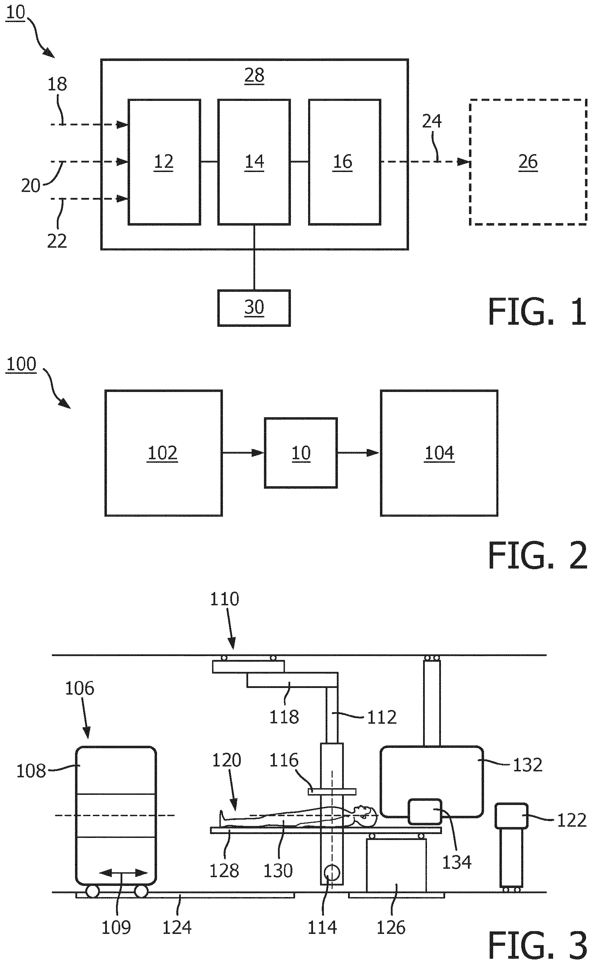

Fig. 1 schematically shows an example of a device 10 for positioning an imaging device in a dual-modality imaging system. The device 10 comprises a data input 12, a data processor 14 and an output interface 16. The data input 12 is configured to receive first image data 18 of a subject from a first X-ray imaging modality. The first image data 18 is indicated by a first arrow in dotted lines. The data input 12 is also configured to receive at least one procedural parameter 20 relating to a current interventional medical procedure of the subject. The at least one procedural parameter 20 is indicated by a second arrow in dotted lines. The data input 12 is further configured to provide a spatial registration 22 of the first X-ray imaging modality and a second X-ray imaging modality. The spatial registration 22 is indicated by a third arrow in dotted lines. The second X-ray imaging modality is different than the first X-ray imaging modality. The data processor 14 is configured to determine anatomical parameters of the subject in the first image data. The data processor 14 is also configured to calculate positional data 24 for the second X-ray imaging modality for generating current image data as second image data of the subject with the second imaging modality. The positional data 24 is based on the determined anatomical parameters, the at least one procedural parameter 20 and the provided spatial relation 22. The output interface 16 is configured to provide the calculated positional data 24 for generating the second image data of the subject by the second X-ray imaging modality. The calculated positional data 24 is indicated by a fourth arrow in dotted lines.

As an option, the second X-ray imaging modality is indicated with a dotted frame 26.

A further frame 28 indicates a common housing for the data input 12, the data processor 14 and the output interface 16. In another option, the components are provided separately.

The term "data input" relates to providing or supplying data for data processing steps. The data input can also be referred to as image data input. The data input can also be referred to as data supply, as image data supply, as image input, as input unit or simply as input. In an example, the image data input is data-connectable to an imaging source arrangement.

The term "data processor" relates to a processor or part of a processor arrangement that is provided to conduct the computing steps using the data supplied by the data input. The data processor can also be referred to as data processing arrangement, as processor unit or as processor. In an example, the data processor is data-connected to the data input and the output interface.

The term "output interface" relates to an interface for providing the processed or computed data for further purposes. The output interface can also be referred to as output or output unit. In an example, the output interface is data-connectable to a display arrangement or display device. In another example, the output is data-connected to a display. As an example, the signals by the controller can be provided by the output interface.

The image data from the first imaging modality can also be referred to as first image data.

In an example, the first image data is registered to a common reference system of the first and the second imaging modality.

The spatial registration can also be referred to as known spatial relation.

For example, in case where one or more CT scans are made during the procedure, these can be used as roadmap during an angiographic intervention or during a CT guided intervention. As an example, the angiographic system only is used to place the for example the catheter.

In another option, if pre-op data is available, the first CT scan can be omitted to save patient dose, or the scan range can be limited, and the pre-op data is used instead. However, a spatial registration is necessary to link the pre-op data into the same refence system as the first and second imaging modality.

According to an aspect, possible positions of the C-arm and table of the angiographic system are calculated from a scout scan of the CT system. As an example, four previews are provided to the operator which can accept one option to automatically position the C-arm and table.

The calculation of the possible positions is based on the scout scan and the selected procedure, for example a liver embolization. As an option, the CT scanner recognizes the anatomical areas on the scout scan. The position of this anatomical area on the scout scan can be transferred into a C-arm position and a table position. In case of pre-op data, the first CT scan can be omitted, and the pro-op data initial position is calculated based on the segmentation results of the pre-op data and the anatomical regions on the scout scan.

As an example, four positions can be calculated for different arrangement scenarios: In a first position, no table movement is provided, while the C-arm is positioned in an A-P view on a selected anatomical area. In a second position, no table movement is provided, and the C-arm is positioned only in a lateral view on the selected anatomical area. In a third position, the table is moved to a configured position and the C-arm is positioned in the A-P view on the selected anatomical area. In a fourth position, the table is moved to a configured position and the C-arm is positioned in the lateral view on the selected anatomical area.

As an effect, the usability of an Angio-CT hybrid system is improved by providing positioning of the C-arm and the table.

In an option, automatic positioning of the C-arm and table is provided based on the CT scout scan. In a further option, automatic calculation of the initial position of the pre-op data is provided based on the CT scout scan.

The Angio-CT system coordinate system is calibrated. Therefore, the position of the CT images in relation of the angiographic system are known. With this information, the positions of the C-arm and table can be calculated.

The calculation of the initial position of the segmented pre-op data is done by shifting the pre-op data in such way that for example the segmented liver in the pre-op data has the same position as the liver anatomical area on the scout scan. This is only required if the initial CT scan is skipped otherwise, we could calculate the position of the pre-op data based on a 3D-3D registration.

A calculated initial position of the pre-op data is within the capture range of the 2D/3D registration method.

A user interface may be provided that gives the possibility to position the angiographic system based on the scout scan, for example in an automatic manner.

Further examples for the field of use are percutaneous needle guidance and embolization procedures.

The term "procedural parameter" relates to the current procedure which provides the context in which the second images are planned to be taken. The at least one procedural parameter reflects the medical situation of the subject and provides sufficient information to know the basic criteria for the upcoming imaging by the second imaging modality. The information thus relates to imaging parameters such as possible and suitable viewing directions in relation to the subject's anatomy, field of view, brightness, contrast, energy range and the like.

The term "spatial registration" relates to a known relative spatial arrangement of the first and second imaging modality. This allows to register the image data from the first imaging modality into the spatial frame or reference system of the second imaging modality.

The term "anatomical parameters" relates to determination of basic anatomical structures within the first image to allow a basic spatial registration of the subject to the spatial frame or reference system of the first imaging modality.

The term "positional data" relates to a relative positioning of subject and the second imaging modality for the purpose of acquiring the second images of the subject.

According to an aspect, the first and second imaging modality both operate in the same spatial reference system. Images from the first imaging modality are taken as a basis to identify the subject and the respective anatomical structure. Based on the known spatial relation of the first and the second imaging modality, the position data for the second imaging modality can be calculated and provided for the user for acquiring the second type of images.

In an option of Fig. 1, the first image data 18 is X-ray CT imaging data. The second image data is X-ray C-arm imaging data, and the positional data comprises position data of an X-ray C-arm imaging device serving as the second imaging modality.

As an effect, positioning the angiographic system in an angio-CT suite is facilitated even if the angiographic system moves over larger distances to be able to perform CT acquisition like images.

In an option, the C-arm imaging is provided with an at least partly contrast-injected anatomical structure.

In another option, the C-arm imaging is provided with a contrast-free anatomical structure.

In a further option, the C-arm imaging is provided as fluoroscopy imaging.

In an option of Fig. 1, the first image data 18 is used for generating navigational information used during the current interventional medical procedure.

For example, the navigational information is provided as a roadmap. The first image data can also be referred to as scout image. The scout image can be provided as pre-operational image data or as intra-operational image data. In case of intra-operational image data, a spatial registration of the image data and the first imaging modality is provided.

In an option of Fig. 1, the at least one procedural parameter 20 comprises information about a selected or current medical procedure.

For example, the medical procedure is a certain imaging procedure. In another example, the medical procedure is an interventional procedure, such as a catheterization, ablation, biopsy or implantation procedure. The interventional procedure can also be an examination procedure, such as intraluminal imaging procedures.

The medical procedure can also be referred to as clinical procedure. An example for a clinical procedure is liver embolization. The second X-ray images are provided to monitor the procedure.

The procedural parameter as functional parameter thus provides the regime for defining the imaging position(s) of the second imaging modality.

In an option of Fig. 1, the calculated positional data 24 for the second imaging modality comprises at least one imaging position for an imaging procedure according to the defined procedural parameter.

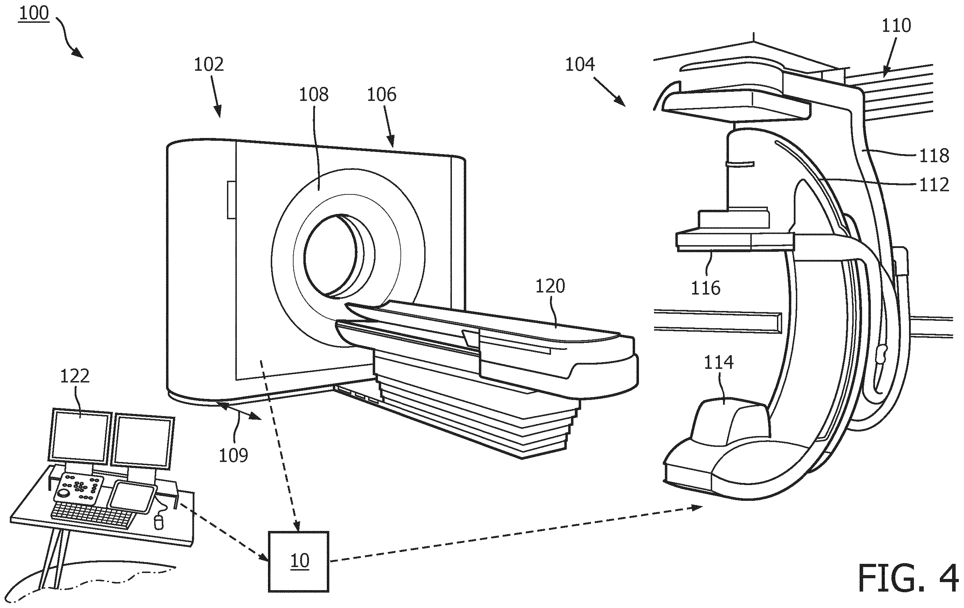

In an option of Fig. 1, the calculated positional data 24 comprises at least two imaging positions for an imaging procedure according to the defined procedural parameter. A user interface (see e.g. console in Fig. 4) is provided configured to receive a selection of one of the least two imaging positions and providing the selected imaging position to the second X-ray imaging modality.

In an option of Fig. 1, a database 30 is provided with stored predefined optimized viewing directions for selected medical procedures and selected anatomical scenarios. The database 30 can be connected to the data processor 14 as shown, or also to the data input 12. An example are imaging directions for certain needle or device movement directions.

In an example, the predefined optimized viewing directions are customizable by a user. In an option of Fig. 1, the anatomical parameters are determined based on a segmentation of the first image data. As an option, the data processor 14 is configured to segment the first image data.

Fig. 2 shows an example of a medical dual-modality imaging system 100 for generating first and second type of X-ray image data of a subject. The system 100 comprises a first X-ray imaging modality 102, a second X-ay imaging modality 104 and an example of the device 10 for positioning an imaging device in a dual-modality imaging system according to one of the examples above. The first X-ray imaging modality 102, and the second X-ray imaging modality 104 provide different image acquisition techniques. The first X-ray imaging modality 102 provides the first image data of the subject. The second X-ray imaging modality 104 is configured to generate the second image data based on the calculated positional data.

Lines with arrows indicate a data connection of the first X-ray imaging modality 102, the device 10 for positioning an imaging device in a dual-modality imaging system and the second X-ray imaging modality 104.

The system 100 is provided for generating first and second type of X-ray image data of a subject in an alternating manner.

Fig. 3 shows another example of the medical dual-modality imaging system 100. The first imaging modality 102 is an X-ray CT imaging device 106 with a movable gantry 108; the second imaging modality 104 is a C-arm X-ray imaging device 110 with a movably mounted C-arm 112.

A double arrow 109 indicates the movability of the gantry 108.

As an example, an X-ray source 114 and an X-ray detector 116 are mounted to opposing arms of the C-arm 112. A support structure 118 may be provided suspending from a ceiling. Also wall- or floor-mounted support structures can be provided.

In an option of Fig. 3, a common spatial coordinate system (not shown in detail) is provided to which the X-ray CT imaging device 106 and the C-arm X-ray imaging device 110 are linked.

In an option of Fig. 3, a movable subject support 120 is provided. For the relative positioning of the second imaging modality, the subject support 120 is configured for a movement of the subject when the subject is arranged on the subject support 120.

As another option, a console 122 is provided for controlling the various equipment in the arrangement. The console 122 may provide one or several of the group of displays, touch pads, keyboard, mouse, graphic tablet, control knobs and the like.

As an option, rails 124 are indicated for the CT gantry 108. The subject support 120 may be provided with a fixed base 126 and a movable support surface 128. Further, a subject 130 is indicated. A display arrangement 132 is shown together with a further control interface 134.

Fig. 4 shows a perspective view of another example of the medical dual-modality imaging system with an example of the X-ray CT imaging device 106 and an example of the C-arm X-ray imaging device 110.

As an example, the X-ray C-arm imaging 110 device is provided as an angiographic system providing angiographic image data.

In an option, the first image data provides 2D information about a region of interest of the subject. Based on the known geometry of the acquisition systems and subject support, the longitudinal position can be derived from the 2D information, and the lateral and frontal position can be derived from the known geometries with a sufficient accuracy.

As an example, a normal scout-scan provides 2D information. As another example, also an X-ray image provides 2D information.

As a further example, a frontal and a lateral scout-scan, or frontal and lateral X-ray images, provide more information.

The accuracy can be increased if 3D information is available.

In another option, the first image data provides 3D information about a region of interest of the subject.

As an example, the first image data provided by the X-ray CT imaging device comprises spatial information due to imaging from different angles.

In an alternative option (not shown in detail), the first X-ray imaging modality is an X-ray C-arm imaging device and the image data from the first imaging modality is X-ray C-arm image data; the positional data comprises position data of an X-ray CT imaging device serving as the second imaging modality.

As another example, the first image data provided by the X-ray C-arm imaging device comprises at least two images acquired as bi-lateral images with two different projection directions, such as orthogonal to each other.

As an option, the position data of an X-ray CT imaging device can be set such that the imaging size or field size of the X-ray CT imaging device is reduced to a minimum. This supports in the general goal to reduce X-ray radiation dose for the subject as well as for the staff members. It also provides reduced time and means facilitation by providing an improved ease of use.

The term "linked" refers to a known spatial relationship such that the relative positions of both imaging devices is preferably always available.

As an example, the X-ray CT imaging device and the C-arm X-ray imaging device are referring to the common spatial coordinate system. Both imaging devices are located within the common spatial coordinate system and provide image data relating to the common spatial coordinate system.

As another example, a local spatial reference system of the X-ray CT imaging device and a local spatial reference system C-arm X-ray imaging device are linked to the common spatial coordinate system.

The position of the CT images in relation to the angiographic C-arm system are thus well defined.

The spatial coordinate system can also be referred to as common spatial reference system or common reference.

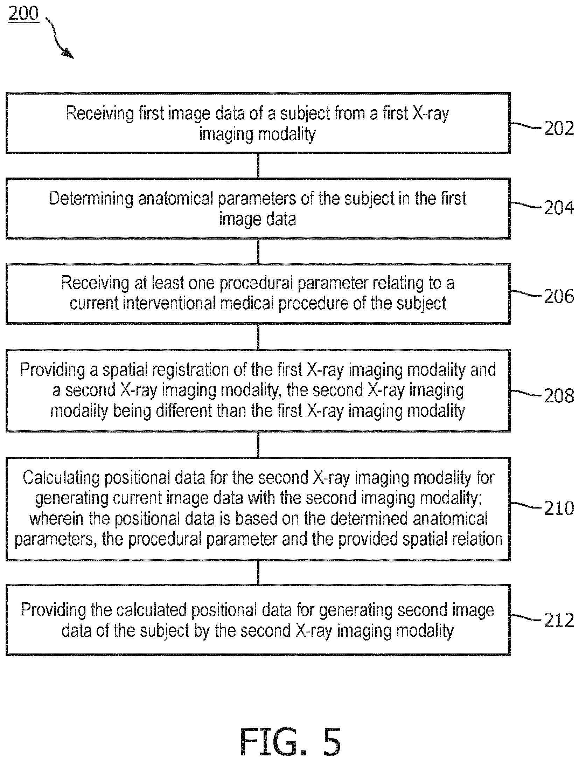

Fig. 5 shows basic steps of an example of a method 200 for determining imaging parameters. The method comprises the following steps: In a first step 202, first image data of a subject from a first X-ray imaging modality is received. In a second step 204, anatomical parameters of the subject are determined in the first image data. In a third step 206, at least one procedural parameter relating to a current interventional medical procedure of the subject is received. In a fourth step 208, a spatial registration of the first X-ray imaging modality and a second X-ray imaging modality is provided, wherein the second X-ray imaging modality is different than the first X-ray imaging modality. In a fifth step 210, positional data for the second X-ray imaging modality for generating current image data with the second imaging modality is calculated. The positional data is based on the determined anatomical parameters, the procedural parameter and the provided spatial relation. In a sixth step 212, the calculated positional data for generating second image data of the subject by the second X-ray imaging modality is provided.

In an example of the method, in an additional step, generating second image data of the subject with the second X-ray imaging modality based on the calculated positional data is provided.

In an example of the method, the first image data is X-ray CT imaging data. The positional data comprises position data of an X-ray C-arm imaging device serving as the second imaging modality. The second image data is X-ray C-arm imaging data.

In an example of the method, the first image data is used for generating navigational information used during the current interventional medical procedure.

In an example of the method, the at least one procedural parameter comprises information about a selected or current medical procedure.

In an example of the method, the calculated positional data comprises at least two imaging positions for an imaging procedure according to the defined procedural parameter. It is further provided the step of selecting one of the least two imaging positions and providing the selected imaging position to the second X-ray imaging modality.

In an example, it is provided to acquire a scout scan with a CT system. Next, a clinical procedure is selected, for example a liver embolization or stent placement. Further, the recognized anatomical areas are read in on the scout scan from the CT. As an option, if the required anatomical area is not recognized or the clinical procedure is not indicated, the user can also manually indicate the anatomical area on the scout scan. As another option, the initial position of the segmented pre-op data is calculated. Furthermore, the possible positions of the C-arm and table are calculated.

According to an aspect, a positioning of an angiographic system and, as an option, pre-op data, is done based on a scout scan of the CT device of the angio-CT system arrangement.

In an example, an angiographic device is provided that comprises a C-arm and that is also equipped with a table, i.e. subject support, which is shared with a CT scanner device.

The CT scanner and the C-arm are provided to be movable in relation to the subject support. In an option, the CT scanner and the C-arm are both movable in a horizontal direction across the room for the acquisition purpose, while the subject support is provided to be fixed in horizontal direction for the acquisition but can be adapted in the vertical direction.

In an example, the subject support can be moved in the horizontal direction but remains fixed between the first images and the second images. This results in an increased accuracy, since table inclination, when moving the table surface horizontally across the stand, is avoided.

The term "subject" may also be referred to as individual. The "subject" may further also be referred to as patient, although it is noted that this term does not indicate whether any illness or disease is actually present with the subject.

In an example, a computer program is provided that comprises instructions which, when the program is executed by a computer, cause the computer to carry out the method of one of the examples above.

In an example, a computer program or program element for controlling an apparatus according to one of the examples above is provided, which program or program element, when being executed by a processing unit, is adapted to perform the method steps of one of the method examples above.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention. Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.
It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for positioning an imaging device in a dual-modality imaging system, the device comprising:
- a data input (12);
- a data processor (14); and
- an output interface (16);
wherein the data input is configured: to receive first image data (18) of a subject from a first X-ray imaging modality; to receive at least one procedural parameter (20) relating to a current interventional medical procedure of the subject; and to provide a spatial registration (22) of the first X-ray imaging modality and a second X-ray imaging modality, the second X-ray imaging modality being different than the first X-ray imaging modality;
wherein the data processor is configured: to determine anatomical parameters of the subject in the first image data; and to calculate positional data (24) for the second X-ray imaging modality for generating current image data as second image data of the subject with the second imaging modality, wherein the positional data is based on the determined anatomical parameters, the procedural parameter and the provided spatial relation; and
wherein the output interface is configured: to provide the calculated positional data for generating the second image data of the subject by the second X-ray imaging modality.

2. Device according to claim 1, wherein the first image data is X-ray CT imaging data; and wherein the second image data is X-ray C-arm imaging data, and the positional data comprises position data of an X-ray C-arm imaging device serving as the second imaging modality.

3. Device according to claim 1 or 2, wherein the first image data is used for generating navigational information used during the current interventional medical procedure.

4. Device according to claim 1, 2 or 3, wherein the at least one procedural parameter comprises information about a selected or current medical procedure.

5. Device according to one of the preceding claims, wherein the calculated positional data for the second imaging modality comprises at least one imaging position for an imaging procedure according to the defined procedural parameter.

6. Device according to one of the preceding claims, wherein the calculated positional data comprises at least two imaging positions for an imaging procedure according to the defined procedural parameter; and
wherein a user interface is provided configured to receive a selection of one of the least two imaging positions and providing the selected imaging position to the second X-ray imaging modality.

7. Device according to one of the preceding claims, wherein a database (30) is provided with stored predefined optimized viewing directions for selected medical procedures and selected anatomical scenarios.

8. Device according to one of the preceding claims, wherein the anatomical parameters are determined based on a segmentation of the first image data; and
wherein the data processor is configured to segment the first image data.

9. A medical dual-modality imaging system (100) for generating first and second type of X-ray image data of a subject, the system comprising:
- a first X-ray imaging modality (102);
- a second X-ay imaging modality (104); and
- a device (10) according to one of the preceding claims;
wherein the first X-ray imaging modality and the second X-ray imaging modality provide different image acquisition techniques;
wherein the first X-ray imaging modality provides the first image data of the subject; and
wherein the second X-ray imaging modality is configured to generate the second image data based on the calculated positional data.

10. System according to claim 9, wherein the first imaging modality is an X-ray CT imaging device (106) with a movable gantry (108); and
wherein the second imaging modality is a C-arm X-ray imaging device (110) with a movably mounted C-arm (112).

11. System according to claim 10, wherein a common spatial coordinate system is provided to which the X-ray CT imaging device and the C-arm X-ray imaging device are linked.

12. System according to one of claims 9 to 11, wherein a movable subject support (120) is provided; and
wherein for the relative positioning of the second imaging modality, the subject support is configured for a movement of the subject when the subject is arranged on the subject support.

13. A method (200) for determining imaging parameters, the method comprising the following steps:
- receiving first image data (202) of a subject from a first X-ray imaging modality;
- determining (204) anatomical parameters of the subject in the first image data;
- receiving (206) at least one procedural parameter relating to a current interventional medical procedure of the subject;
- providing (208) a spatial registration of the first X-ray imaging modality and a second X-ray imaging modality, the second X-ray imaging modality being different than the first X-ray imaging modality;
- calculating (210) positional data for the second X-ray imaging modality for generating current image data with the second imaging modality; wherein the positional data is based on the determined anatomical parameters, the procedural parameter and the provided spatial relation; and
- providing (212) the calculated positional data for generating second image data of the subject by the second X-ray imaging modality.

14. Computer program comprising instructions which, when the program is executed by a computer, cause the computer to carry out the method of claim 13.

15. Computer readable medium having stored the computer program of claim 14.
